# EUROPÄISCHE PATENTSCHRIFT

(11) **EP 1 854 438 B1**
(45) Veröffentlichungstag und Bekanntmachung des Hinweises auf die Patenterteilung: **04.07.2018**
(21) Anmeldenummer: 06009539.5
(22) Anmeldetag: 09.05.2006
(51) Int. Cl.: A61F 9/008

(54) **Ophthalmologische Vorrichtung zur Vorbeugung einer Myopie**
Ophthalmologic device for preventing myopia
Dispositif ophthalmologique pour prévenir de la myopie

(43) Veröffentlichungstag der Anmeldung: 14.11.2007
(73) Patentinhaber: IROC Services AG, 6300 Zug (CH); Koller, Tobias, 8002 Zürich (CH); Seiler, Stefan, 8002 Zürich (CH)
(72) Erfinder: Seiler, Theo, 8002 Zürich (CH); Koller, Tobias, 8002 Zürich (CH); Seiler, Stefan, 8002 Zürich (CH)
(74) Vertreter: von Hellfeld, Axel

(56) Entgegenhaltungen:
- WO-A-90/11054
- WO-A-99/44491
- WO-A-2005/110397
- WO-A2-01/87181
- US-A- 5 445 608
- US-A1- 2003 009 205
- US-B1- 6 443 976
- DATABASE WPI Week 199345 Derwent Publications Ltd., London, GB; AN 1993-358292 XP002404536 & SU 1 771 732 A1 (EYE MICROSURGERY RES TECH INST) 30. Oktober 1992 (1992-10-30)

## Beschreibung

Die Erfindung betrifft eine Vorrichtung zur Vorbeugung einer Fehlsichtigkeit oder Lederhauterkrankung eines Auges.

Bei der sogenannten Myopie oder Kurzsichtigkeit können weit entfernte Objekte schlechter gesehen werden als nahegelegene. Die Myopie bezeichnet eine Form der Fehlsichtigkeit, bei der der Fokus vor der Netzhaut liegt. Dabei werden zwei prinzipielle Formen der Kurzsichtigkeit unterschieden, zum einen die "Brechungsmyopie" bei normaler "Augenachsenlänge, aber zu starker Brechkraft, und die sogenannte "Achsenmyopie" bei normaler Brechkraft, aber zu langer Augenachse. Für die erste Form, die "Brechungsmyopie", kennt der Stand der Technik vielfältige ophthalmologische Vorrichtungen, die der Korrektur dieses Sehfehlers dienen.

Die zweite Form - die "Achsenmyopie" - beschreibt eine häufig auftretende Erkrankung des Auges, bei der das Auge durch (passives) Sklerawachstum länger wird. Dies erfolgt insbesondere in den ersten drei Lebensjahrzehnten. Hierbei kommt es zu einer allmählichen und übermäßigen Verlängerung des Augapfels. Die Ursachen hierfür sind noch nicht gefunden. Es wird angenommen, dass die genetische Veranlagung eine wichtige Rolle spielt.

Derzeit sind drei Formen der "Achsenmyopie" bekannt: die Schulmyopie, die gutartige progressive Myopie und die bösartige progressive Myopie.

Bei der Schulmyopie beginnt der Augapfel sich kurz vor der Pubertät (im Alter zwischen 10 und 12 Jahren) zu strecken und verstärkt die Kurzsichtigkeit kontinuierlich, bis das Körperwachstum mit etwa 20-25 Jahren abgeschlossen ist. Dabei können die Augen z.B. eine Brechkraft von ungefähr minus 6 Dioptrien erreichen.

Bei der gutartigen progressiven Myopie stabilisiert sich die Brechkraft erst mit 30 Jahren. Dann kann die Kurzsichtigkeit bei ungefähr 12 Dioptrien liegen.

Schließlich schreitet bei der bösartigen progressiven Myopie die unregelmäßige Dehnung des Augapfels auch bis ins höhere Alter weiter fort. Dann beginnt der Glaskörper zunehmend an der Netzhaut zu zerren, weil diese nicht mit der Lederhaut (Sklera) mitwächst. Hierdurch steigt das Risiko einer Netzhautablösung.

Stand der Technik findet sich in SU 1771732 A1 und WO 2005/110397 A1, die Einrichtungen zur Behandlung von Myopie offenbaren. Weiterer Stand der Technik findet sich in der US 5,445,608, die ein Verfahren und eine Apparatur zur Bereitstellung einer Licht-aktivierten Therapie beschreibt.

Derzeit kennt die Ophthalmologie kein Mittel, mit dem sich das passive Sklerawachstum aufhalten ließe.

Mit der vorliegenden Erfindung soll eine Vorrichtung bereitgestellt werden, mit der die vorstehend erläuterte Fehlsichtigkeit infolge einer Lederhauterkrankung oder Lederhautveränderung eines Auges behandelt werden kann. Die Erfindung wird in Anspruch 1 dargelegt. Bevorzugte Ausführungsformen der Erfindung werden in den abhängigen Ansprüchen geschildert.

Die Vorrichtung ist gekennzeichnet durch eine Einrichtung zum Verfestigen der sich im hinteren Augenabschnitt befindenden Lederhaut.

Die Verfestigung der Lederhaut erfolgt mit einer erfindungsgemäßen Vorrichtung mit zumindest einer Strahlungsquelle zum Bestrahlen der Lederhaut, wobei die zu verfestigende Lederhaut bevorzugt homogen bestrahlt wird. Eine homogene Bestrahlung mit elektromagnetischen Strahlung liegt dann vor, wenn pro Flächeneinheit im wesentlichen die gleiche Strahlungsmenge auf die Lederhaut trifft. Eine solche homogene Bestrahlung kann mit einer punktförmigen Strahlungsquelle, deren gebündelte Strahlung über die sphärisch gekrümmte Lederhaut geführt wird, erreicht werden. Gemäß einer bevorzugten Ausführungsform kann eine homogene Bestrahlung der Lederhaut auch mit einer Einrichtung erreicht werden, die die Strahlung homogen verteilt. Hierzu sieht die Erfindung bevorzugt besondere Maßnahmen zur Homogenisierung der Strahlungsverteilung vor.

In Abwandlung der vorstehend beschriebenen Ausführungsform der Erfindung ist es auch möglich, Steuereinrichtungen für die Strahlungsverteilung über die Lederhaut so vorzusehen, dass die pro Flächeneinheit auf die Lederhaut auftreffende Strahlungsmenge in Abhängigkeit vom Ort auf der Lederhaut wahlweise einstellbar ist. Bevorzugt erfolgt die Verfestigung von circa 5 mm hinter dem Limbus bis zum hinteren Pol.

Ferner sieht eine besondere Ausgestaltung vor, dass eine Einrichtung vorgesehen ist zur Bestimmung von Eigenschaften der Lederhaut und/oder anderer Komponenten des Auges. Gegebenenfalls können diese Messungen an unterschiedlichen Stellen der Lederhaut zu unterschiedlichen Ergebnissen führen, was wiederum für die oben erwähnte Steuerung der Intensitätsverteilung der elektromagnetischen Strahlung in Abhängigkeit vom Ort des Auges bei besonderen Ausgestaltungen von Bedeutung sein kann.

Die Einrichtung zum Verfestigen der Lederhaut mittels elektromagnetischer Strahlung kann so gestaltet sein, dass sie einen gewählten Abstand von der Lederhaut besitzt oder auf dieser aufliegt, d.h. gemäß einer bevorzugten Ausgestaltung kann die Einrichtung bei bestimmungsgemäßem Gebrauch einen vorgegebenen Abstand von der Kornea besitzen oder mit der Kornea in Kontakt gebracht werden. Des weiteren werden hierin auch unterschiedliche Strahlungsquellen für die elektromagnetische Strahlung und unterschiedliche Techniken beschrieben, diese Strahlung zum Einsatzort zu führen. Einzelheiten hierzu finden sich in der nachfolgenden Beschreibung von Ausführungsbeispielen.

Gemäß einer Variante der Erfindung ist es vorgesehen, die Einrichtung, mit der die elektromagnetische Strahlung auf die Lederhaut gestrahlt wird, mit einem Endoskop auszustatten, das mit einem Operationsmikroskop gekoppelt ist und zwar bevorzugt derart, dass der Operateur während des Aufbringens der elektromagnetischen Strahlung das Auge und insbesondere die Lederhaut oder Teile davon beobachten kann.

Weiterhin wird ein Verfahren zum Vorbeugen einer Fehlsichtigkeit eines Auges infolge einer Lederhauterkrankung beschrieben, bei dem mit elektromagnetischer Strahlung eine Verfestigung der Lederhaut durchgeführt wird. Bei diesem Verfahren wird bevorzugt ein Photomediator eingesetzt, der in der Haut ein sogenanntes "Cross-Linking" bewirkt. Bei dem Verfahren wird die Strahlung bevorzugt homogen auf die Sklera aufgebracht oder aber gemäß einem besonderen Steuerprogramm ungleichmäßig wenn bestimmte Bereiche der Sklera anders bestrahlt werden sollen als andere Bereiche derselben, zum Beispiel aufgrund unterschiedlicher Stärken der Sklera oder aufgrund unterschiedlicher Zielvorgaben für die Verfestigung (das Cross-Linking) in Abhängigkeit vom Ort der Sklera.

Weitere bevorzugte Ausgestaltungen der Erfindung ergeben sich aus den abhängigen Patentansprüchen und der nachfolgenden Beschreibung von Ausführungsbeispielen anhand der Zeichnung. Es zeigt:
- Figur 1: eine Vorrichtung zur Vorbeugung einer Fehlsichtigkeit eines Auges;
- Figur 2: eine abgewandelte Ausführungsform einer Vorrichtung zur Vorbeugung einer Fehlsichtigkeit des Auges;
- Figur 3: eine weitere Ausführungsform einer Vorrichtung zur Vorbeugung einer Fehlsichtigkeit des Auges;

In den Figuren sind einander entsprechende oder funktionsähnliche Bauteile mit gleichen Bezugszeichen versehen.

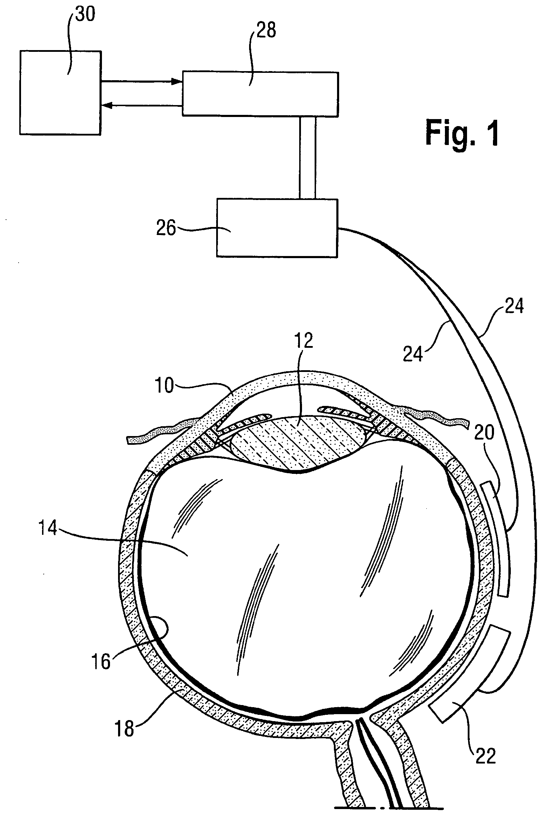

Figur 1 zeigt schematisch ein Auge mit einer Hornhaut 10, einer Linse 12, einem Glaskörper 14, einer Netzhaut 16 und einer Lederhaut (Sklera) 18.

Beim Ausführungsbeispiel nach Figur 1 liegen ein Band 20 und ein Kissen 22 direkt von Außen auf der Lederhaut (Sklera) 18 auf. Das Band 20 und das Kissen 22 sind derart geformt, dass elektromagnetische Strahlung in Richtung auf die Lederhaut 18 gestrahlt werden kann. Dazu können das Band 20 und das Kissen 22 auf der von der Sklera abgekehrten Seite verspiegelt sein.

Faserlichtleiter 24 sind mit dem Band 20 und dem Kissen 22 verbunden. Es ist auch möglich, dass jeweils mehrere Lichtleiterfasern mit dem Band 20 und dem Kissen 22 verbunden sind. Im dargestellten Ausführungsbeispiel werden die Lichtleiterfasern 24 aus einer Strahlungsquelle 26, zum Beispiel einem Laser oder LEDs, die entweder in cw-mode oder gepulst betrieben werden, gespeist. Mittels einer Stormversorgung 28 wird die Strahlungsquelle 26 angesteuert, das heißt, die über die Lichtleiterfasern 24 abgegebene Strahlungsmenge kann selektiv, je nach Bedarf, eingestellt werden. Für die Steuerung der von der Strahlungsquelle 26 abgegebenen Strahlungsmengen ist eine Steuer- und Regeleinheit 30 vorgesehen, die zum Beispiel rechnergesteuert sein kann.

Im Inneren des Bandes 20 und Kissens 22 befindet sich im Strahlengang der von den Lichtleiterfasern 24 abgegebenen Strahlung, ein sogenannter "Diffusor" (nicht gezeigt). Die Funktion des Diffusors ist es, die von den Lichtleiterfasern 24 abgegebene Strahlung möglichst gleichmäßig über die Sklera zu verteilen, sodass Intensitätsspitzen vermieden werden. Zum Beispiel kann der Diffusor als milchglasartig streuendes Material ausgebildet sein. Eine andere Variante der Ausgestaltung des Bandes 20 und des Kissens 22 sieht vor, dass an einem Stirnende des Bandes bzw. des Kissens das Licht eingekoppelt wird und dieses dann auf seinem Laufweg parallel zur Sklera durch gestaffelte Reflexionsflächen sukzessive aus dem Band oder Kissen nach einer Seite austritt, um die Sklera mit homogener fluence (Energie pro Flächeneinheit) zu bestrahlen.

Das Ausführungsbeispiel nach Figur 1 kann auch mit einem Endoskop (nicht gezeigt) ausgestattet werden, das mit einem Operationsmikroskop (nicht gezeigt) gekoppelt ist. Dieses kann mit einem Filter versehen sein, das ermöglicht, dass der Operateur ohne Beeinträchtigung durch die von der Strahlungsquelle 26 und über die Leiterfaser 24 geleitete elektromagnetische Strahlung, einerseits die interessierenden Augenteile beobachten kann und andererseits die Diffusoren unter Sicht richtig positioniert werden können.

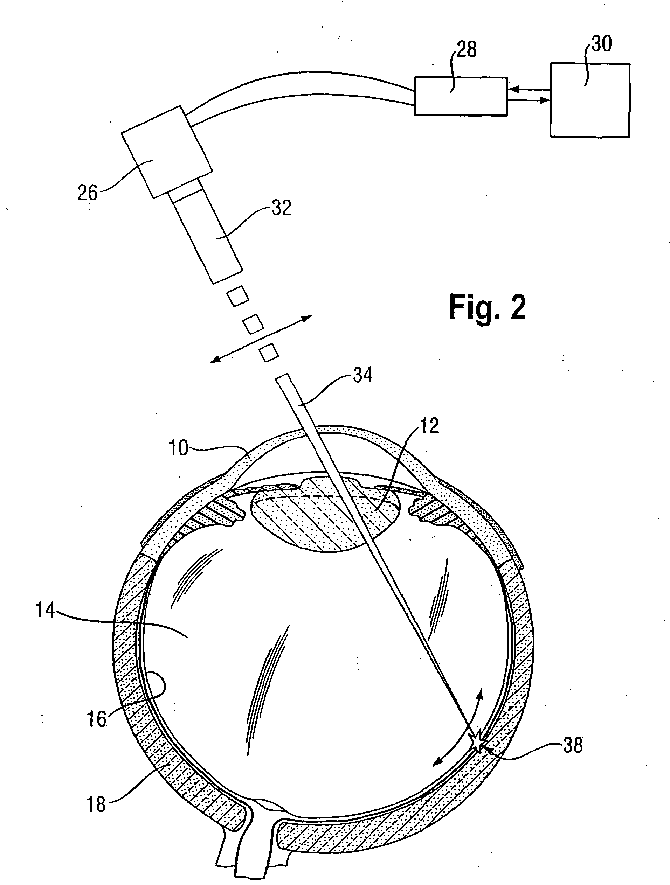

Figur 2 zeigt eine Abwandlung der Behandlung der Lederhaut 18 mit elektromagnetischer Strahlung. Bei dem Ausführungsbeispiel nach Figur 2 wird elektromagnetische Strahlung über das Innere des Auges durch die Hornhaut 10, die Linse 12, den Glaskörper 14 und die Netzhaut 16 auf die Lederhaut 18 appliziert. Dabei tritt gebündelte Strahlung 34 aus einer Strahlungsfokussierungseinrichtung 32 aus, die mit einer Strahlungsquelle 26 verbunden ist. Die gebündelte Strahlung 34 trifft unter Berücksichtigung von Effekten, die innerhalb des Auges auftreten können und eine Veränderung der Strahlungsrichtung bewirken können, auf die Lederhaut 18 in einem Punkt 38 auf. Um eine homogene Bestrahlung der Lederhaut 18 zu bewirken, wird die gebündelte Strahlung 34 derart über die Lederhaut 18 geführt, dass pro Flächeneinheit im wesentlichen die gleiche oder eine gewünschte, örtlich variierende Strahlungsmenge auf die Lederhaut trifft. Dieses "Abscannen" der Lederhaut 18 mit Strahlung 34 erfordert eine Bewegung der Strahlung relativ zur Lederhaut. Dies kann zum Beispiel mit einem Spiegel (nicht gezeigt) in an sich bekannter Weise durchgeführt werden. Diese Bewegung der Strahlung ist in Figur 2 mit Pfeilen angedeutet.

Die Strahlungsquelle 26, die die Strahlungsfokussierungseinrichtung 32 speist, wird mittels einer Stromversorgung 28 angesteuert. Die von der Strahlungsquelle 26 abgegebene Strahlungsmenge kann selektiv, je nach Bedarf eingestellt werden. Für die Steuerung der von der Strahlungsquelle 26 abgegebenen Strahlungsmenge ist eine Steuer- und Regeleinheit 30 vorgesehen, die zum Beispiel rechnergesteuert sein kann.

Während einer Bestrahlung können mehrere Strahlungsquellen 26 und oder Strahlungsfokussierungseinrichtungen 32 eingesetzt werden, um eine Bestrahlung der Lederhaut 18 an mehreren Stellen gleichzeitig zu bewirken. Dies hätte zum Beispiel den Vorteil, dass eine Behandlung verkürzt, oder dass die Bestrahlung der Lederhaut symmetrisch ausgeführt werden könnte.

Zusätzlich kann ein Strahlungssensor (nicht gezeigt) vorgesehen sein, der einen Teil der in Richtung auf die Lederhaut gerichteten Strahlung erfasst. Das Messsignal des Sensors wird über eine Leitung in die Steuer- und Regeleinheit zur Verarbeitung übertragen, damit die Steuer- und Regeleinheit entsprechend die Stromversorgungseinheit für die Strahlungsquelle bzw. die Strahlungsquellen ansteuern kann. Dabei ist bevorzugt vorgesehen, dass jede einzelne Strahlungsquelle selektiv ansteuerbar ist, so dass für die einzelnen Strahlungsquellen unterschiedliche Strahlungsintensitäten vorgesehen werden können.

Zusätzlich kann ein als solches bekannter sogenannter "Justierstrahl" für die Positionierung des Auges eingesetzt werden. Ein solcher Strahl wird in der Literatur auch bisweilen als "Fixierlichtstrahl" bezeichnet. Damit lässt sich die Positionierung des Auges in Bezug auf die beschriebenen Vorrichtungen und den Laserstrahl 34 verbessern. Es ist auch möglich, die hier beschriebenen Vorrichtungen mit einem sogenannten "eye-tracker" zu kombinieren. Solche "eye-tracker" sind Augenverfolgungssysteme, die mögliche Bewegungen des Auges optisch verfolgen und für die Chirurgie eingesetzten Instrumente, zum Beispiel Laserstrahlen, entsprechend der Augenbewegung nachführen.

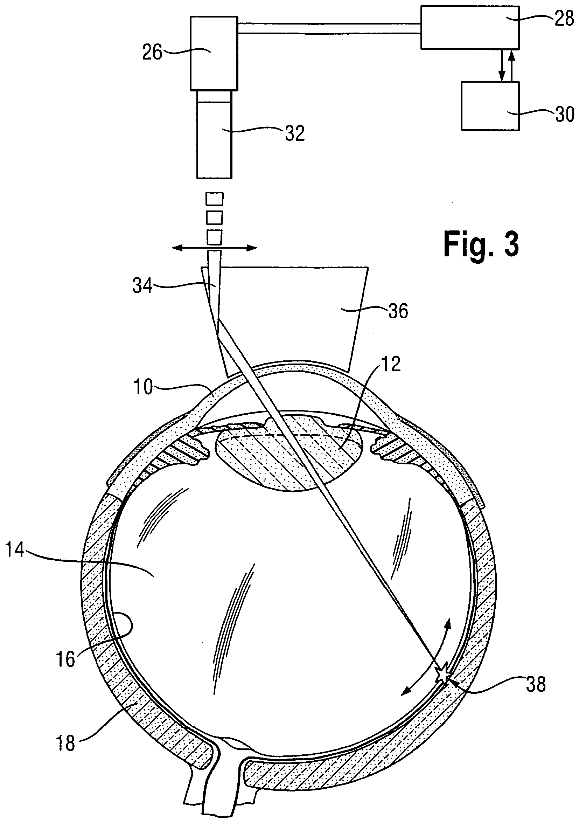

Figur 3 zeigt eine weitere Vorrichtung zur Bestrahlung der Lederhaut 18. Bei diesem Ausführungsbeispiel befindet sich zwischen der Strahlungsfokussierungseinrichtung 32 und der Hornhaut 10 eine Einrichtung 36, die dazu dient, eine gezielte Änderung der Strahlungsrichtung der Strahlung 34 herbeizuführen. Mit der Einrichtung 36, die direkt auf oder nahe der Kornea positionierbar ist, ist es möglich, den Einstrahlwinkel der auf die Sklera gerichteten Strahlung so einzustellen, dass Strahlung bis sehr nahe an den Limbus herangeführt werden kann.

Es ist, gemäß einer anderen Variante auch möglich, die Positionierung der beschriebenen Vorrichtungen und Einrichtungen am Auge, mit einem Brillengestell zu fördern.

Mit den anhand der Figuren 1, 2 und 3 erläuterten Ausführungsbeispielen der Erfindung ist es möglich, die Lederhaut 18 zu festigen. Hierfür kann vor der Behandlung ein Photomediator auf die Lederhaut 18 homogen oder gezielt aufgebracht und eingebracht werden. Die Bestrahlung wird mit Infrarot-Wellenlängen oder Wellenlängen im sichtbaren optischen Bereich durchgeführt. Als Photomediator dienen insbesondere Bengalrosa oder andere Substanzen, die im optischen Wellenlängenbereich oder im nahen Infrarot absorbieren. Dabei setzt der Photomediator Radikale frei, die ihrerseits neue molekulare Bindungen erzeugen und somit das Gewebe festigen ("crosslinking", "x-linking"). Die Strahlungsquelle 26 ist hierfür entsprechend ausgelegt. Es ist auch möglich, eine Verfestigung der Lederhaut ohne Photosensibilisator nur durch die Strahlung selbst durchzuführen. Jedoch muss hierbei darauf geachtet werden, dass die Netzhaut 16 nicht durch kurzwellige Strahlung geschädigt wird.

Die Verfestigung der Lederhaut mit Vorrichtungen gemäß den Figuren 1, 2 und 3 kann durch den Einsatz von besonderen Messungen am Auge verbessert werden.

So ist es zum Beispiel mit Mitteln, die der Strand der Technik bereithält, möglich, die Lederhautdicke und Lederhautfestigkeit optisch oder akustisch zu bestimmen. In Abhängigkeit von der so ermittelten Lederhautdicke oder anderen Parametern können dann die Prozessparameter (Strahlungsmenge, Photomediator) hinsichtlich der Verfestigung der Lederhaut 18, wie oben beschrieben eingestellt werden.

Es ist auch möglich, zusammen mit den gezeigten Vorrichtungen die Mikroskopie zur Beurteilung von eventuell auftretenden Gewebeeffekten einzusetzen, um unerwünschte Störungen zu vermeiden. Auch kann während des Einsatzes der Vorrichtung vorgesehen sein, den Augeninnendruck zu bestimmen, um daraus gegebenenfalls Steuergrößen für die Verfestigung der Lederhaut 18 abzuleiten. Entsprechendes gilt für den Einsatz von bekannten Verfahren für die optische Spektroskopie zur Gewebecharakterisierung oder auch Verfahren, die mittels akustooptischer Spektroskopie eine Gewebecharakterisierung ermöglichen.

## Patentansprüche

1. System zum Verfestigen der Lederhaut des Auges mittels Bestrahlung der Lederhaut mit Licht, wobei zur Bestrahlung der Lederhaut (18) ein Band (20) und/oder Kissen (22) eingerichtet ist, zur Strahlungsverteilung von außen auf die Lederhaut aufgelegt zu werden, wobei Band und Kissen derart geformt sind, dass das Licht in Richtung auf die Lederhaut gestrahlt wird,
wobei das Band (20) und das Kissen (22) gestaffelte Reflexionsflächen umfassen, und
wobei das System derart eingerichtet ist, dass an einem Stirnende des Bandes beziehungsweise des Kissens Licht einkoppelbar ist und das Licht auf seinem Laufweg im Wesentlichen parallel zu einer Oberfläche der Lederhaut durch die gestaffelten Reflexionsflächen sukzessive aus dem Band beziehungsweise dem Kissen nach einer Seite zur Lederhaut hin zur Bestrahlung der Lederhaut austreten kann, um das Licht möglichst gleichmäßig über die Lederhaut zu verteilen.

2. System nach Anspruch 1, **dadurch gekennzeichnet, dass** Lichtleiterfasern (24) vorgesehen sind, Licht einer Strahlungsquelle (26) zu Band (20) und Kissen (22) zu übertragen.

3. System nach einem der vorhergehenden Ansprüche, mit einem Endoskop, das mit einem Operationsmikroskop gekoppelt ist.

4. System nach einem der vorhergehenden Ansprüche, **dadurch gekennzeichnet, dass** sie eingerichtet ist, die Verfestigung durch Licht von circa 5 Millimeter hinter dem Limbus bis zum hinteren Pol des Auges durchzuführen.

## Claims

1. A system for strengthening the sclera of the eye by irradiating the sclera with light, wherein for the irradiation of the sclera (18), a strip (20) and/or pad (22) are/is configured for being placed on the sclera from the outside in order to distribute the radiation, wherein the strip and the pad are shaped in such a way that the light is radiated in the direction of the sclera,
wherein the strip (20) and the pad (22) include graduated reflective surfaces, and
wherein the system is configured in such a way that light may be coupled in at an end-face side of the strip or the pad, and the light on its path may successively exit, essentially in parallel to a surface of the sclera, through the graduated reflective surfaces and from the strip or the pad to one side toward the sclera in order to irradiate the sclera, so that the light is distributed over the sclera as uniformly as possible.

2. The system according to Claim 1, **characterized in that** optical fibers (24) are provided for transmitting light of a radiation source (26) to the strip (20) and the pad (22).

3. The system according to one of the preceding claims, including an endoscope that is coupled to a surgical microscope.

4. The system according to one of the preceding claims, **characterized in that** it is configured for carrying out the strengthening, using light, from approximately 5 millimeters behind the limbus to the posterior pole of the eye.

## Revendications

1. Système pour consolider la sclérotique de l'oeil au moyen d'une irradiation de la sclérotique avec de la lumière, dans lequel, pour l'irradiation de la sclérotique (18), une bande (20) et/ou un coussin (22) est/sont aménagé(e)s afin d'être placé(e)s sur la sclérotique par l'extérieur en vue de la répartition du rayonnement, la bande et le coussin étant formés de telle sorte que la lumière est irradiée en direction de la sclérotique,
dans lequel la bande (20) et le coussin (22) comprennent des surfaces réfléchissantes échelonnées, et
dans lequel le système est aménagé de telle sorte que la lumière peut être injectée à une extrémité frontale de la bande ou du coussin, et que la lumière sur sa trajectoire substantiellement parallèle à une surface de la sclérotique peut successivement sortir à travers les surfaces réfléchissantes échelonnées de la bande ou du coussin vers un côté de la sclérotique en vue de l'irradiation de la sclérotique afin de répartir la lumière de manière aussi homogène que possible sur la sclérotique.

2. Système selon la revendication 1, **caractérisé en ce que** des fibres optiques (24) sont prévues pour transmettre la lumière d'une source de rayonnement (26) à la bande (20) et au coussin (22).

3. Système selon l'une quelconque des revendications précédentes, avec un endoscope couplé à un microscope opératoire.

4. Système selon l'une quelconque des revendications précédentes, **caractérisé en ce qu'**il est aménagé pour effectuer la consolidation par la lumière de 5 millimètres derrière le limbe jusqu'au pôle postérieur de l'oeil.
